(19)

Europäisches
Patentamt

European
Patent Office

Office européen
des brevets

(11)　 **EP 2 637 991 B1**

(12)　　　　　　　　　**EUROPEAN PATENT SPECIFICATION**

(45) Date of publication and mention
of the grant of the patent:
**07.08.2019 Bulletin 2019/32**

(21) Application number: **11778547.7**

(22) Date of filing: **14.10.2011**

(51) Int Cl.:
***C07C 29/151*** *(2006.01)*

(86) International application number:
**PCT/EP2011/068013**

(87) International publication number:
**WO 2012/062529 (18.05.2012 Gazette 2012/20)**

(54) **METHOD AND APPARATUS FOR THE CARBON DIOXIDE BASED METHANOL SYNTHESIS**

VERFAHREN UND VORRICHTUNG FÜR METHANOLSYNTHESE AUF KOHLENDIOXIDBASIS

PROCÉDÉ ET APPAREIL DE SYNTHÈSE DE MÉTHANOL À BASE DE DIOXYDE DE CARBONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2010 PCT/EP2010/067182**

(43) Date of publication of application:
**18.09.2013 Bulletin 2013/38**

(73) Proprietor: **Silicon Fire AG
6045 Meggen (CH)**

(72) Inventor: **MEYER-PITTROFF, Roland
85354 Freising (DE)**

(74) Representative: **Heusch, Christian
Ant-IP GmbH
Kirchplatz 2
82387 Antdorf (DE)**

(56) References cited:
**WO-A2-00/25380　　　　WO-A2-2008/012039
DE-A1-102009 007 567**

- **"Basisdaten Biogas Deutschland", , 31 March
2005 (2005-03-31), pages 1-7, XP55028350,
Germany Retrieved from the Internet:
URL:http://wiki.istanbullisesi.net/images/
9/96/Basisdaten_Biogas.pdf [retrieved on
2012-05-29]**
- **OLAH GEORGE A: "Beyond oil and gas: the
methanol economy.", ANGEWANDTE CHEMIE
(INTERNATIONAL ED. IN ENGLISH), vol. 44, no.
18, 29 April 2005 (2005-04-29), pages 2636-2639,
XP002677487,**
- **OLAH G A; GOEPPERT A; PRAKASH G K S:
"Chemical recycling of carbon dioxide to
methanol and dimethyl ether: From greenhouse
gas to renewable, environmentally carbon
neutral fuels and synthetic hydrocarbons",
JOURNAL OF ORGANIC CHEMISTRY, vol. 74, no.
2, 16 January 2009 (2009-01-16), pages 487-498,
XP002677488,**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

The present invention concerns a method and an apparatus for the synthesis of methanol based on carbon dioxide and hydrogen.

The present application claims the priority of the international patent application with the application number PCT/EP2010/067812, which was filed on 10 November 2010 and which carries the title "METHOD AND APPARATUS FOR THE INTEGRATED SYNTHESIS OF METHANOL IN A PLANT". This international patent application concerns a methanol synthesis being fully integrated into an overall system. Coal or hydrocarbon is combusted in a combustion chamber together with enriched oxygen gas. The oxygen gas is provided by a water electrolyzer. Carbon dioxide is fed into a reforming system after it has been washed out of the flue gas produced by the combustion process. The reforming system produces a synthesis gas. The respective synthesis gas essentially consists of carbon monoxide and hydrogen. Methanol is then produced using the synthesis gas plus additional hydrogen provided by the water electrolyzer.

The hydrogen economy is by some experts believed to have the potential to replace essentially the fossil fuel economy. The introduction of the hydrogen economy is regarded to have the potential to cut carbon dioxide emissions and to reduce the dependence on fossil fuels.

However, methanol ($CH_3OH$) is regarded to be far more convenient than the very light, reactive and volatile hydrogen. One methanol molecule "carries" four hydrogen atoms which makes methanol a promising hydrogen carrier. Methanol is at normal conditions a liquid which burns clean and requires only minor modifications to existing fuel-delivery infrastructure and to combustion engines. If the synthesis of methanol would make use of carbon dioxide, the carbon dioxide footprint could be reduced.

A number of projects are known concerning various aspects of the carbon dioxide based methanol synthesis. Experiments have revealed that the catalyzers, which are used for synthesizing methanol, are very sensitive to impurities. If the carbon dioxide is to be taken from the flue gas of a conventional power plant or combustion engine, there are a number of impurities and contaminants which would have to be removed. Typical flue gas washing solutions which are used for the sequestration of carbon dioxide in large scale systems, are not able to provide carbon dioxide in a form which is clean enough for use in a subsequent methanol synthesis reactor. The sequestration of carbon dioxide has the additional disadvantage that it consumes quite some energy.

It is known to produce methanol based on a synthesis gas which in this case comprises carbon dioxide and hydrogen, as presented in the following equation [1]:

$$CO_2 + 3H_2 \rightarrow CH_3OH + H_2O \text{ (-49.6 kJ/mol at 298 K).} \qquad [1]$$

The equation [1] shows an exothermic reaction, i.e. a reaction which releases energy. The main components of a corresponding synthesis plant, such as the commercially available Silicon Fire Mobile Station™ offered by the applicant, are a synthesis reactor and a distillation column having the required assemblies, as well as measuring and regulating units.

The synthesis gas could come from various sources, as long as it has a certain purity grade dictated mainly by the catalyzer used in the synthesis reactor. The required carbon dioxide until now is typically transported to the synthesis reactor preferably liquefied under adequate conditions (e.g. at approx. -23 °C and 18-20 bar pressure) and is temporarily stored in a carbon dioxide tank.

The document DE102009007567A1 discloses a method and apparatus for the production of methanol by reducing with hydrogen carbon dioxide contained in the flue gas of a power plant using inter alia biogas as a fuel. This document further discloses to use hydrogen from a water electrolysis and to co-feed the oxygen into the biogas.

G.A. Olah discloses details about the Methanol economy in the publication "Beyond Oil and Gas: The Methanol Economy", Angewandte Chemie Int. Ed., 2005, Vol. 44, pages 2636-2639. He specifically mentions the production of methanol by reacting $H_2$ with $CO_2$ from industrial effluents or the atmosphere.

In the technical paper "Chemical Recycling of Carbon Dioxide to Methanol and Dimethyl Ether: From Greenhouse Gas to Renewable, Environmentally Carbon Neutral Fuels and Synthetic Hydrocarbons", by G.A. Olah et al, Journal of Organic Chemistry, 2009, Vo. 74, No. 2, pages 487-498, a method for the chemical recycling of carbon dioxide is disclosed. The respective process disclosed, enables the production of methanol and/or dimethyl ether (DME).

A system and method for the storage of electrical energy or of hydrogen is disclosed in WO0025380 A2. The respective method comprises the steps of (a) electrolysis of water to yield hydrogen (b) reaction of the hydrogen from step (a) with carbon dioxide to form at least one storage compound; (c) storage of this storage compound; and (d) subsequent conversion of this storage compound back into hydrogen or use of this storage compound to fuel an internal combustion engine or to generate electricity either directly or indirectly. A preferred storage compound mentioned in WO0025380 A2 is methanol.

Methane is the major component of all important gaseous combustion gases. It is for instance a predominant component of natural gas as well as mine gas. Biogenic combustion gases, such as biogas, swamp gas, fermentation gas, dump gas, sewage or digester gas comprise about 60 vol.-% methane. In addition, the biogenic combustion gases comprise carbon dioxide, vapor ($H_2O$) and small amounts of by-products, such as hydrogen sulfide ($H_2S$) and ammonia ($NH_3$). Biogenic combustion gases are generated when organic material is microbially broken down. Organic matter can be defined as all substances of herbal or animal origin with high carbon content.

The hydrogen required for the synthesis of methanol can be delivered in gaseous form in bundles of gas cylinders or liquefied in cryogenic tanks. Likewise, the hydrogen can be generated at the synthesis plant itself with the aid of an electrolysis plant by splitting water in accordance with equation [2]:

$$H_2O \text{ (liquid)} \rightarrow H_2 + 0.5O_2 \text{ (+286.02 at 298 K).} \qquad [2]$$

The reaction [2] is quite energy consuming, and the hydrogen is very light and volatile and thus difficult to store and to transport, as already mentioned.

Most combustion processes employ oxygen contained in air. Air comprises about 79 vol.-% nitrogen ($N_2$) and only about 21 vol.-% oxygen ($O_2$). Due to this composition of air, the flue gas of a combustion process comprises nitrogen, too. The combustion is prone to producing undesired by-products, especially nitrogen oxides (NOx).

If methane is combusted with oxygen only, as presented in equation [3], carbon dioxide $CO_2$ and water $H_2O$ are produced:

$$CH_4 + 2O_2 \rightarrow CO_2 + 2H_2O \text{ (liquid) (-889 kJ/mol = -55,56 MJ/kg = gros calorific value)} \qquad [3]$$

There are no undesired by-products if the feed-gas on the left-hand side of equation [3] is "clean". The reaction of equation [3] produces $CO_2$ and $H_2O$.

It is also known in the art to run a combustion process with an increased oxygen content. The required oxygen can be provided by a water electrolysis, as disclosed in the patent US 5,342,702 with title "Synergistic process for the production of carbon dioxide using a cogeneration reactor". The patent US 5,342,702 mentions the possibility to produce methanol using some of the $CO_2$ produced as by-product of a main process which uses a feed stream of organic combustible fuel and hydrogen.

It is also known in the art to generate power by an oxygen-enriched combustion of coal in combination with the $CO_2$-based synthesis of methanol. The respective process is disclosed in WO 95/31423 with title "Production of methanol". According to this patent application, DC power from a photovoltaic system is used to supply a water electrolysis system. The hydrogen is used for the methanol production. The oxygen may be used to feed the combustion process.

Another process is disclosed in WO 2008/012039, with title "Verfahren zur Reduzierung der CO2-Emission fossil befeuerter Kraftwerksanlagen", where hydrogen is obtained electrolytically.

Combustion processes are currently being tested in pilot plants where an air-separation step is carried out to separate nitrogen and oxygen. An oxygen-rich gas is then fed into a combustion zone. It is an advantage of this approach that the combustion is more efficient since it takes place at higher temperatures and produces flue gas with less nitrogen. It is, however, a disadvantage that the air-separation plant requires investment and operational costs and that it consumes energy.

It is known in the art to generate power in combination with the sequestration of $CO_2$-emissions. A respective process, disclosed in patent US 6,148,602, with title "Solid-fueled power generation system with carbon dioxide sequestration and method therefore", includes the compression of ambient air, the separation of pure oxygen from the ambient air and as a further step the compression of the oxygen separated from the ambient air. After the oxygen has been further compressed, the oxygen is divided into a first oxygen stream and a second oxygen stream. The first oxygen stream and a solid fuel, such as coal, are fed into a solid-fuel gasifier for converting the first oxygen stream and the solid fuel into a combustible gas. The gas is then combusted in the presence of the second oxygen stream.

The $CO_2$ produced in a combustion process has to be separated out if the $CO_2$-emission of the respective plant should be reduced by a post-processing of the $CO_2$. The flue gas containing the $CO_2$ typically also contains nitrogen, dust, sulfur oxides, water vapor and other constituents or components. Fossil power plants thus require a special sequestration system for separating the $CO_2$ from the rest of the flue gas constituents or components. The respective washing process currently used consumes quite some energy, as mentioned before. This means that a significant proportion of the energy produced by a fossil power plant is to be re-invested in the $CO_2$ sequestration. The cleaner the combustion process is and the higher the concentration of $CO_2$ is, the easier and more efficient is the respective $CO_2$-sequestration process. In this respect it is advantageous to run a combustion process so that it is close to the pure oxygen-based combustion of equation [3]. The pure oxygen-based combustion is herein referred to as "clean" combustion.

The final form of energy from renewable sources is in most cases electric energy. For instance wind farms, solar plants and hydropower plants typically generate electric energy. The electric energy could be used to drive auxiliary units of the $CO_2$-sequestration facilities, or the electric energy could be used to drive the above-mentioned air-separation. These approaches are, however, not regarded to be very promising. The invention therefore uses a different approach.

It is known in the art to produce hydrocarbons from hydrogen and $CO_2$. If one would take the $CO_2$ from a power plant flue gas, the energetic efficiency and the cleaning capability of the sequestration process are essential. In order to efficiently produce methanol, the stoichiometric composition of the reactants has to be proper, as shown in the above equation [1]. The synthesis of 1 mol of $CH_3OH$ requires 3 mol $H_2$ and 1 mol $CO_2$.

It is an objective of the present invention to provide an improved method and an apparatus for the synthesis of methanol based on carbon dioxide and hydrogen. The focus is on an improved overall efficiency and a careful use of resources.

It is an objective of the present invention to provide an improved method and an apparatus for the synthesis of methanol based on carbon dioxide and hydrogen which is at least to some degree independent from external supplies.

Summary of the invention:

The invention relates to

1) a method for the generation of methanol (116) and for providing output power (E5, E7, E8) in a plant (100), comprising the process steps:- carrying out a water electrolysis process (106) producing oxygen gas (101) and hydrogen gas (107),- providing a combustion gas composition (CGC) comprising at least 40 vol.-% hydrocarbon gas (102) and at least 25 vol.-% carbon dioxide (117),-at an input side (201) of a combustion chamber (200), feeding said combustion gas composition (CGC) and said oxygen gas (101) into the combustion chamber (200),- maintaining an oxygen-based combustion process (103) for the combustion of the combustion gas composition (CGC) in said combustion chamber (200) in order to provide output power (E8), said combustion process (103) releasing a flue gas (104) at an output side (204) which contains more than 65 vol.-% carbon dioxide (109),- combining (41) said carbon dioxide (109) and said hydrogen gas (107) to form a gas mixture,-feeding said gas mixture into a catalytic reactor (220),- in said catalytic reactor (220) carrying out a catalytic process (114) which processes said gas mixture in order to provide methanol (116), wherein at least part of said flue gas (104) or of said carbon dioxide (109) is fed back from said output side (204) to said input side (201) of the combustion chamber (200) in order to increase the amount of carbon dioxide (109) at the input side (201), and wherein means (111, 205) for removing water (H2 0) from said flue gas (104) are employed; and

2) a plant (100) for the generation of methanol (116) and for providing output power (E5, E7, E8) comprising:- a water electrolysis facility (210) which can be supplied by electric energy (E4) and water (105) and being designed in order to produce hydrogen gas (107) and oxygen gas (101), the water electrolysis facility (210) comprising a hydrogen gas outlet (211) and an oxygen gas outlet (212),-a thermal engine with at least one combustion chamber (200) designed for maintaining an oxygen-based combustion process (103) in order to provide output power (E8), said combustion chamber (200) comprising an input side (201), and a flue gas outlet (204) for releasing a flue gas (104) which contains more than 65 vol.-% carbon dioxide (109),- a gas connection (250) for feeding said oxygen gas (101) from said oxygen gas outlet (212) to the input side (201) of the combustion chamber (200),-a gas connection (202) for feeding a combustion gas composition (CGC) comprising a hydrocarbon gas (102) and carbon dioxide (117) to the input side (201) of the combustion chamber (200),- a gas mixer (41; 53) for providing a gas mixture, said gas mixer (41; 53) being connectable to said hydrogen gas outlet (211) and being directly or indirectly connectable to said flue gas outlet (204),- a catalytic reactor (220) for carrying out a catalytic process (114) which processes said gas mixture in order to provide said methanol (116),- a combustion gas-oxygen mixer or a gas-oxygen-CO2 mixer (207) at the input side (201) of the combustion chamber (200),- further comprising means (111, 205) for removing water (H2O) from said flue gas (104).

According to the invention, one process step is the "clean" combustion of a hydrocarbon gas, such as natural gas or biogenic gas. According to the invention, a combustion gas composition is employed which comprises at least 35 vol.-% hydrocarbon gas and at least 15 vol.-% carbon dioxide. The "clean" combustion requires the supply of pure oxygen gas having an oxygen concentration of at least 75 vol.-%. The corresponding combustion (oxidation) of the combustion gas composition with pure oxygen is described in equations [3.1] and [3.2]. The "clean" combustion process has the advantages that on the one hand the combustion as such is more efficient, if an adequate combustion chamber (optionally with flue gas recirculation and/or high-temperature resistant materials, coatings, overlay or layer) is used which is designed to correspond with the significant higher combustion temperature (to withstand temperatures between 800 and 2000 °C, depending on the kind of combustion chamber). According to the invention, the flue gas contains a very high $CO_2$-concentration and no or only very few unwanted contaminants and impurities as by-products. This fact makes a direct supply to a subsequent $CO_2$-based methanol synthesis process feasible and robust.

According to the invention, a further process step is the catalytic synthesis of methanol, as described by equations [1], [1.1] and [1.2]. The respective synthesis consumes synthesis gas essentially consisting of carbon dioxide and hydrogen. This synthesis is carried out using the ideal or close-to-ideal stoichiometric ratio of reactants in a very pure form. The synthesis gas preferably has a mixture with a ratio of 1 mol of carbon dioxide per 3 mol of hydrogen.

According to the invention, a further process step is the electrolytic splitting of water (hereinafter called water electrolysis). The water electrolysis provides hydrogen and oxygen, as illustrated by equations [2], [2.1] and [2.2]. Preferably, all embodiments are designed so as to produce (exactly) the amount of hydrogen required for establishing the synthesis gas mixture, because the production of excess hydrogen would lead to a reduced overall efficiency.

Furthermore, at least part of the flue gas or of the carbon dioxide is fed back from an output side of the combustion chamber to an input side of the combustion chamber in order to increase the amount of carbon dioxide at the input side, and means for removing water from the flue gas are employed.

In order to optimize the synthesis process, the water electrolysis and the clean combustion process in respect to the energetic balance and also to the reaction conditions (e.g. to avoid the formation of critical contaminants and impurities) are combined as presented by the following exemplary matrix:

$$4CO_2 + 12H_2 \rightarrow 4CH_3OH + 4H_2O \qquad [1.1]$$

$$12H_2O \text{ (liquid)} \rightarrow 12H_2 + 6O_2 \qquad [2.1]$$

$$3CH_4 + CO_2 + 6O_2 \rightarrow 4CO_2 + 6H_2O \qquad [3.1]$$

The reaction in accordance with equation [3.1] employs as an example a combustion gas composition with 25 vol.-% $CO_2$ and 75 vol.-% $CH_4$. All of the oxygen gas of the water electrolysis (see equation [2.1]) is employed in the reaction of equation [3.1] for combustion purposes. The reaction of equation [3.1] produces 4 mol of carbon dioxide. This carbon dioxide together with the hydrogen produced by the water electrolysis (see equation [2.1]) serve as synthesis gas. Equation [1.1] shows that this synthesis gas can be used to produce 4 mol of methanol plus 4 mol water.

If, according to the invention, the combustion gas composition is a physical mixture of 40 vol.-% $CO_2$ and 60 vol.-% $CH_4$, the optimized processes are combined as presented by the following matrix:

$$8CO_2 + 24H_2 \rightarrow 8CH_3OH + 8H_2O \qquad [1.2]$$

$$24H_2O \text{ (liquid)} \rightarrow 24H_2OH + 12_2 \qquad [2.2]$$

$$6CH_4 + 4CO_2 + 12O_2 \rightarrow 10CO_2 + 12H_2O \qquad [3.2]$$

The reaction [3.2] employs a combustion gas composition with a higher $CO_2$ concentration (40 vol.-%) than in the case of the reaction [3.1]. Hence, the reaction [3.2] produces more $CO_2$ than the reaction [3.1]. All of the oxygen gas of the water electrolysis (see reaction [2.2]) is employed in reaction [3.2] for combustion purposes. Reaction [3.2] produces 10 mol of carbon dioxide. 80% of this carbon dioxide together with the hydrogen produced by the water electrolysis (see equation [2.2]) serve as synthesis gas. Equation [1.2] shows that this synthesis gas can be used to produce 8 mol of methanol plus 8 mol water. Please note that the reaction [3.2] produces more $CO_2$ than required or consumed by the reaction [3.2]. The excess $CO_2$ can be put into a buffer tank for further use.

In a preferred embodiment of the invention, the electric energy consumed by the water electrolysis is at least to some extent provided from local or remote renewable sources. Most preferred is an embodiment where all of the electric energy for the water electrolysis is renewable.

Some of the electric energy might be provided by the "clean" combustion process, the combustion chamber of which is part of a gas and /or steam power plant where an electric generator is driven by a gas and/or steam turbine. The combustion chamber can also be a part of a thermal engine which drives an electric generator.

The above process steps, which so far were regarded as individual, unrelated steps, according to the present invention form a nearly ideal process matrix for the efficient production of methanol. The expression "matrix" is herein used to emphasize the fact that the above-mentioned process steps are not coupled one after the other in a linear process chain. Instead the processes are intertwined and dependent on each other.

It is a special advantage of this process matrix that the methanol so produced is to some extent renewable and that at the same time it is $CO_2$-neutral since $CO_2$ emissions from a clean combustion process are consumed.

It is a further advantage of the present invention that the oxygen from the electrolysis (cf. reactions [2.1] or [2.2]) is used in the process matrix in order to feed or drive the clean combustion process (reactions [3.1] or [3.2]).

The inventive process matrix is regarded to define a synergistic process where all reactants are constituents of a stoichiometrically optimized setup.

The present invention relates to an integrated process matrix for producing energy (electric energy and/or heat) and methanol. The term "integrated" is herein used to define a process matrix where all three process steps of the matrix are directly connected or linked concerning the material flows and the energy flows (electric energy and/or heat).

The integrated nature of the inventive process matrix becomes visible if the respective main equations are listed together (see above equations [1.1] - [3.1] or [1.2] - [3.2]). These equations are written in a form considering the respective molarities so that the overall process becomes an integrated process with balanced molarities.

The inventive process matrix is regarded to be a kind of a cogenerating process matrix since it produces in the first place methanol and in the second place provides output power (electric energy and/or heat) from the clean combustion of the hydrocarbon gas (reaction [3.1] or [3.2]), from the water electrolysis (lost heat from reactions [2.1] or [2.2]) and from the methanol synthesis (excess heat from reactions [1.1] or [1.2]).

In preferred embodiments, the hydrocarbons (preferably methane) are employed in order to provide some of the energy which is required for the splitting of water (reaction [2.1] or [2.2]). CH4 is an example for a gaseous hydrocarbon. Other hydrocarbons or carbon containing fuels (like alcohols) could be used instead or in addition.

The $CO_2$ and the hydrocarbons (preferably methane) together serve as carbon sources for the production of methanol. All of the hydrocarbons (preferably methane) are transformed into $CO_2$ and $H_2O$ which can be removed easily by condensation. The $CO_2$ together with pre-existing $CO_2$ is then used to synthesize the methanol.

The use of gaseous hydrocarbons has advantages. Preferred embodiments consume gaseous hydrocarbons (preferably methane).

It is an advantage of the invention that the flue gas emitted by the clean combustion contains almost only $CO_2$. This $CO_2$, after having prepared the right synthesis gas mixture together with hydrogen, is employed for synthesizing methanol. There are no impurities of the flue gas which would inactivate the catalyzer required for the methanol synthesis.

In preferred embodiments, the energy (heat) of the exothermic process step [3.1] or [3.2] is used, after transformation into electric energy, to a large extent in the endothermic electrolysis process step [2.1] or [2.2].

Also, the reaction and/or loss heats from the methanol synthesis (steps [1.1] or [1.2]) can be used within the power plant cycle and/or for preheating of reaction gases like the combustion oxygen, the methane and carbon dioxide of the combustion gas composition and/or the synthesis gas for the synthesis process.

The process integration is also achieved by using the $CO_2$ of the clean combustion process step (steps [3.1] or [3.2]) as component of the synthesis gas.

According to the invention, the $CO_2$ is not regarded to be a waste product or an undesired gas component. It is used by employing it in the synthesis process (steps [1.1] or [1.2]) together with the hydrogen gas for the production of methanol.

In a preferred embodiment, suitable storages for the needed and produced agents as well as for the heat from the process(es) can be provided at least for the demand of several hours, so that the above mentioned reactions and related processes can run time wise intermittent and with variable load to optimize the economic output.

Preferably, the water electrolysis process (steps [2.1] or [2.2]) is carried out when electric excess energy is available (e.g. during low load times or if excess regenerative energy is available).

Thus, the present invention enables completely new economically and ecologically meaningful solutions for the production of methanol, which can be renewable, as well as for the equalizing of the load fluctuations and the frequency control of electric grids by corresponding control of the electrolyzer's electric consumption.

In a preferred embodiment an energy-integrated overall process matrix is realized using a combination of control hardware and software. The overall energy consumption can be minimized by tuning the process conditions of the exothermic and endothermic reactions. The plant design of a preferred embodiment results in a combination of

- a water electrolysis facility supplied with electric energy and water. The water electrolysis facility is designed in order to produce hydrogen gas and oxygen gas. It comprises a hydrogen gas outlet and an oxygen gas outlet.
- a combustion chamber designed for an oxygen-based combustion process in order to provide heat. The combustion chamber comprises

    ◦ an input side, and
    ◦ a flue gas outlet for releasing a flue gas which contains more than 65 vol.-% carbon dioxide.

- a gas connection for feeding the oxygen gas from the oxygen gas outlet of the electrolysis facility to the input side of the combustion chamber.
- a gas connection for feeding a hydrocarbon-comprising combustion gas composition to the input side of the combustion chamber.
- a catalytic reactor for carrying out a catalytic process which processes a gas mixture comprising the carbon dioxide and the hydrogen gas in order to provide methanol.
- a gas mixer for providing the gas mixture. The gas mixer is connectable to the hydrogen gas outlet of the electrolysis facility and directly or indirectly to the flue gas outlet of the combustion chamber.

According to the invention, the material utilization of the above integrated reaction matrix [1.1] - [3.1] is nearly 100 % and the matrix [1.2] - [3.2] is close to 100 %. The commercial value of natural gas and carbon dioxide is elevated. This means that the mass and energy balances are optimized. The nearly 100 % material utilization is to be calculated over a certain time span. In a real-time set up, where no substantial buffer capabilities are employed, a nearly 100 % atom utilization is given at any point in time. In an embodiment where buffer capabilities are employed, the nearly 100 % atom utilization is ensured over a certain time span only. In the context of the present invention "nearly 100 %" is used for a range between 90 % and 100 %, or preferably between 95 % and 100 %.

In a preferred embodiment of the invention the hydrogen and/or carbon dioxide is stored in dedicated buffer tanks. The size or capacity of these tanks is chosen so that the methanol synthesis plant can run in a constant or near constant mode. This is preferred since this part of the overall plant is expensive and difficult to operate in part load. The corresponding capital investment is only meaningful if the methanol synthesis runs in a constant or almost constant mode.

It is a special advantage of the invention, that a combustion gas composition (e.g. a biogenic gas) which comprises methane and carbon dioxide is combusted together with oxygen so as to produce a relatively clean flue gas. This flue gas, which mainly consists of carbon dioxide, is "recycled" in that it is used for synthesizing methanol. In preferred embodiments, biogenic gas, as emitted by a natural process, is turned (by means of oxidation) into carbon dioxide and some water. The respective carbon dioxide is used for the production of methanol.

The inventive approach provides homogeneous conditions so that the clean combustion process emits no unwanted constituents or by-products, such as NOx, CO, or sooth.

It is a special advantage of the invention, that the combustion engine can be operated in an ideal operational range. This also leads to an improved efficiency and to a very stable and predictable quality of the flue gas.

According to the invention, the biogenic combustion gas composition has a composition as listed below:

| | |
|---|---|
| Methane ($CH_4$): | 40 - 75 vol.-% |
| Carbon dioxide ($CO_2$): | 25 - 55 vol.-% |
| Hydrogen sulfide ($H_2S$): | 10-30000 mg/m$^3$ |
| Ammonia ($NH_3$): | 0,01 - 2,5 mg/m$^3$ |
| Water ($H_2O$): | 0 - 10 vol.-% |
| Nitrogen ($N_2$): | 0,01 - 5 vol.-% |
| Oxygen ($O_2$): | 0,01 - 2 vol.-% |
| Hydrogen ($H_2$): | 0 - 1 vol.-% |

The hydrogen component of the biogenic combustion gas could be separated prior to the clean combustion to be used in the methanol synthesis.

It is a special advantage of the invention, that it provides for a thermodynamically efficient use of the combustion gas composition.

Further details and advantages of the present invention are described in the following on the basis of exemplary embodiments.

Various aspects of the present invention are schematically illustrated in the figures of the drawing:

Figure 1:     shows a functional diagram of the process steps of a first embodiment of the present invention;

Figure 2:     shows a functional hardware diagram of the first embodiment;

Figure 3:     shows a matrix of a first example;

Figure 4:     shows a matrix of a second example.

The term "combustion gas composition" CGC is herein used to describe a gas which comprises a combustible hydrocarbon gas (preferably methane) and $CO_2$. The word "composition" is used to describe a physical mixture of the hydrocarbon gas (preferably methane) and $CO_2$ components.

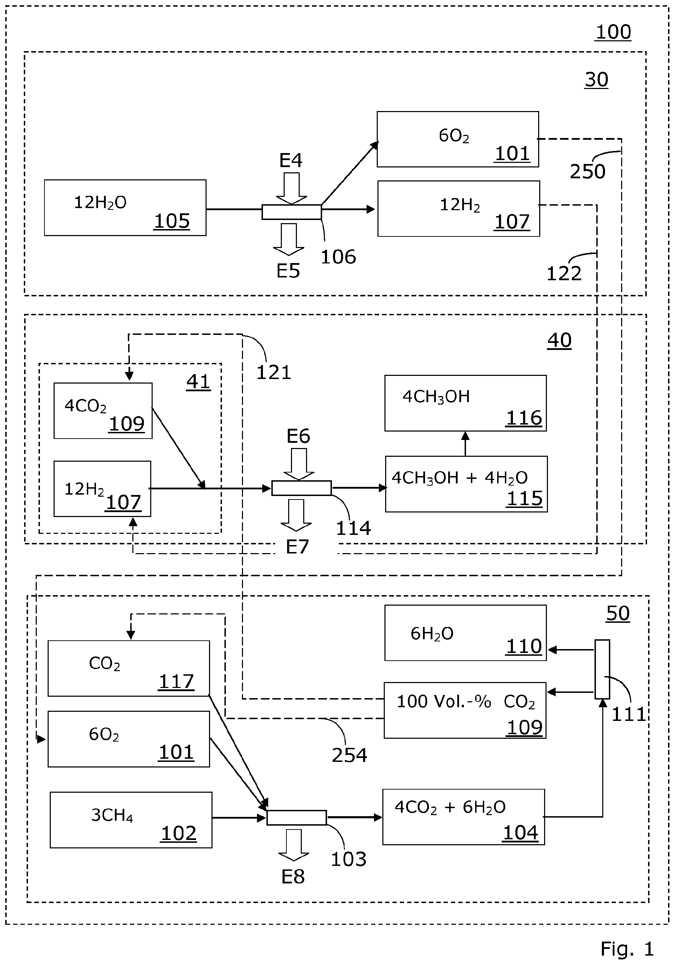

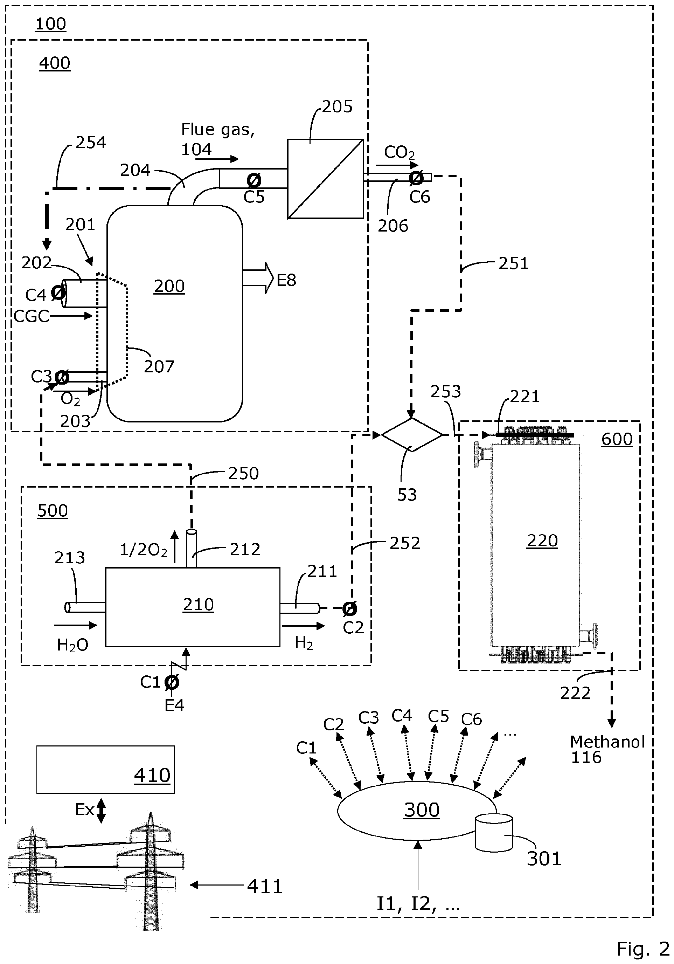

Basic aspects of the invention are addressed and described in connection with Figures 1 and 2.

According to the invention, a water electrolysis process 106 is carried out as one process module 30. The water electrolysis process 106 produces oxygen gas 101 and hydrogen gas 107, as schematically illustrated in Fig. 1 and Fig. 2. The respective electrolyzer 500 comprises a water supply 213 for the infeed of liquid water 105 (or 213). It further comprises a hydrogen gas outlet 211 and an oxygen gas outlet 212. The respective molarities are shown in equations [2.1] or [2.2], and the masses or volumes can be calculated based on these equations. E4 in Fig. 1 and 2 is the electric energy consumed by the water electrolysis process 106 or electrolyzer 500, respectively. As shown in Fig. 2, the respective electrolyzer 500 might be controlled by a control signal C1. The control signal C1 could be a simple on/off signal for switching the electrolyzer 500 on and off, as needed. Since most of the commercially available electrolyses 500 are not designed for an on/off operation, in most practical implementations the control signal C1 is used to adjust the operation of the electrolyzer 500 in a range between 10% and 100% load. Fig. 1 indicates that the electrolyzer 500 emits excess heat (E5 in Fig. 1). As shown in Fig. 2, a control signal C2 could be used to control the hydrogen flow at the hydrogen gas outlet 211.

Preferably, all embodiments comprise a buffer tank (not shown) for storing hydrogen gas 107. During periods where there is not sufficient or sufficiently low-cost electric energy available to run the water electrolysis process 106, the hydrogen gas 107 could be retrieved from the buffer tank.

In a preferred embodiment of the invention, the electric energy E4 consumed by the water electrolysis 106 is at least to some extent provided from (local or remote) renewable sources.

Most preferred is an embodiment where all of the electric energy E4 for the water electrolysis 106 is renewable. Some of the electric energy E4 might be provided by means of the clean combustion process 103. In an ideal set-up of the plant 100, about 10% to 20% of the consumption of electric energy E4 of the module 30 can be covered by electric energy E8 provided by the clean combustion process 103.

According to the invention, the oxygen gas 101 (i.e. a gas comprising more than 75 vol.-% oxygen) is fed to the input side 201 of a combustion chamber 200.

Preferably, all embodiments comprise a combustion gas-oxygen mixer (if the combustion gas composition CGC comprises sufficient $CO_2$) or a gas-oxygen-$CO_2$ mixer 207 (if some of the $CO_2$ produced by process 103 is fed back (see feedback 254) for reasons of temperature control) at the input side 201 of the combustion chamber 200.

According to the invention, a clean combustion process 103 is carried out as one process module 50. In order to maintain a clean, oxygen-based combustion process 103, the combustion chamber 200 is fed at the input side 201 with a hydrocarbon-comprising combustion gas composition CGC (preferably methane 102 plus $CO_2$ 117) and with the oxygen gas 101. The respective gas flows can be controlled using control signals C3 and C4, for example. The combustion of the combustion gas composition CGC in the combustion chamber 200 releases a flue gas 104 at an output side 204 which contains more than 65 vol.-% carbon dioxide 109. The clean combustion is an exothermic process (see equation [3.1] or [3.2] which means that the process releases energy E8 in the form of heat. The heat can be transferred to a nearby site where it could be used for heating purposes (e.g. in the form of steam or hot water), for instance. In most embodiments, at least some of the heat is converted into electric energy by the means of a thermal engine and a generator of the plant 100 (not shown). The electric energy can be used to supply at least some of the energy demand of the water electrolyzer 210.

The clean combustion 103 produces or emits a flue gas 104 which contains a very high volume percentage of $CO_2$ and, depending on the implementation, some water in vapor form. In those cases where water is present, a separation 111 (see Fig. 1) of water 110 and $CO_2$ 109 is carried out. The flue gas 104 - after the removal of water 110 - is fed into a gas mixer 53 (mixing process 41 in Fig. 1). The gas mixer 53 is designed in order to provide a gas mixture via a feed line 253 with the required molarities of $CO_2$ and $H_2$. The respective molarities are shown in equations [1], [1.1] and [1.2] and the masses or volumes can be calculated based on these equations.

Preferably, all embodiments comprise valves or switches which can be controlled by control signals C2 and/or C6 in order to control the supply of $CO_2$ and $H_2$ to the gas mixer 53, as illustrated in Fig. 2. The valves or switches could also be integrated into the gas mixer 53.

Preferably, all embodiments comprise a valve, flap or switch which can be controlled by a control signal C5 in order to control the flue gas 104, as illustrated in Fig. 2.

Preferably, all embodiments comprise a water separator 205 in order to remove water from the flue gas 104, as illustrated in Fig. 2. The process carried out by the water separator 205 is depicted in Fig. 1 as box 111.

In another process module 40, the gas mixture provided by the gas mixer 53 is fed via the feed line 253 into a catalytic reactor 220. Inside this reactor 220 a catalytic process 114 is carried out in order to provide a methanol-water mixture 115 or methanol 116 at an output 222.

According to the invention, the synthesis 114 is carried out using the ideal or close-to-ideal stoichiometric ratio of reactants 109 and 107 in a very pure form. The dashed lines 121, 122 in Fig. 1 indicate that $CO_2$ 109 is provided by the combustion process 103 and that the hydrogen gas 107 is provided by the electrolyzer 210. The dashed lines 121, 122 in Fig. 1 correspond to the connections 251 and 252 in Fig. 2, respectively.

Preferably, all embodiments comprise a catalytic reactor 220 with a ring supply line 221 at the input side. Inside the catalytic reactor 220 there are a number of parallel reactor sections or chambers (not visible in Fig. 2 since they are positioned inside the reactor 220), all of which have to be fed with the same quantity of the gas mixture. The ring supply line 221 ensures the even distribution of the gas mixture into the parallel reactor sections or chambers. Details regarding this aspect of the invention are described in the international patent application PCT/EP2010/064948, which was filed on 6 October 2010.

Preferably, all embodiments comprise a gas feedback 254, as depicted in Fig. 2. The gas feedback 254 is designed in order to feed at least part of the flue gas 104 from the output side 204 of the combustion chamber 200 back to the input side 201. At the input side 201 the flue gas 104 is mixed with the oxygen gas 101 or with the combustion gas composition CGC. It is the main purpose of this gas feedback 254 to keep the temperature inside the combustion chamber 200 within a predefined temperature range from 800 to 2000 °C, depending on the kind of combustion chamber. Since the respective $CO_2$ would appear on both sides of the equations [3.1] and [3.2], the respective terms are not shown in these equations.

Preferably, in all embodiments the combustion chamber 200 is part of an thermal engine. Preferably, in all embodiments a gas Otto engine or a gas diesel engine serves as thermal engine. The thermal engine can be a "component" of a combined heat and power plant (CHP) 400.

For the purposes of the present invention a gas Otto engine is an thermal engine with spark-ignition, designed to run on a combustion gas composition CGC. The gas Otto engine might be an engine which is specifically designed and made for the combustion of gas, or it might be a modified petrol or gasoline engine. In any case, the gas Otto engine comprises, instead of the conventional carburetor, a gas-oxygen mixer or a gas-oxygen-$CO_2$ mixer 207 at the input side 201 of the combustion chamber 200. A respective mixer 207 is indicated in Fig. 2 at the input side 201. Not all embodiments require such a mixer 207, but it is advantageous to equip all embodiments with a respective mixer 207.

If the gas-oxygen stream or the gas-oxygen-$CO_2$ stream has a volume of a few $m^3/h$, small gas Otto engines are very well suited. At higher volume flows pilot injection gas engines, derived from diesel engines, as well as large gas Otto engines can be used.

Since according to the invention the combustion gas composition CGC in any case comprises hydrocarbon (e.g. methane) gas and at least 25 vol.-% $CO_2$, the compression ratio of the engine can be increased compared to combustion gases without $CO_2$. The $CO_2$ gas is considered to be an inert gas which does not actively "participate" in the combustion process 103. This increase of the compression ratio leads to an improved thermal efficiency.

If the invention comprises preferably a combustion engine as thermal engine 400 which has at least two cylinders, only a part of the cylinders of the combustion engine 400 can be fed with the oxygen gas 101, and the other part of the cylinders can run in the traditional way with air as oxygen supplier . Then the flue gases of the differently operated cylinders have to be collected separately. Thus, a greater flexibility is reached concerning the engine's size and the use of combustion gas and oxygen.

Preferably, all embodiments of the invention comprise a combustion engine 400 with an injection cooler for the combustion gas mixture, preferably after a supercharger. A cooling liquid (e.g. the output liquid 115 of the synthesis reaction 114) comprising methanol and water is injected or sprayed into the gas flow prior the combustion chamber 200 for cooling purposes. This increases output and efficiency and decreases the combustion temperature of the engine 400.

Preferably, all embodiments of the invention comprise a combustion engine 400 with an injection cooler. A cooling liquid (e.g. the output liquid 115 of the synthesis reaction 114) comprising methanol and water is combined with or injected or sprayed into the part of the flue gas 104 which is fed back via a feedback line 254 from the output side 204 to the input side 201 of the combustion chamber 200. This helps to increase output and efficiency and to decrease the combustion temperature of the engine 400.

All embodiments may comprise a combustion chamber 200 which is protected by means of a high-temperature anti-corrosion coating, overlay or layer.

The process steps 103, 106 and 114 (process modules 50, 30 and 40), which so far were regarded as individual steps, according to the present invention form a nearly ideal process matrix for the efficient production of methanol 116 and output energy E5, E7, E8. The above-mentioned process steps 103, 106 and 114 (process modules 50, 30 and 40) are intertwined and dependent on each other.

If a combustion gas composition CGC with 75 vol.-% CH4 and 25 vol.-% $CO_2$ is employed, the following equations are valid:

$$4CO_2 + 12H_2 \rightarrow 4CH_3OH + 4H_2O \qquad [1.1]$$

$$12H_2O \text{ (liquid)} \rightarrow 12H_2 + 6O_2 \qquad [2.1]$$

$$3CH_4 + CO_2 + 6O_2 \rightarrow 4CO_2 + 6H_2O \qquad [3.1]$$

These equations [1.1] - [3.1] can be rewritten in form of a table (table 1). This table has a left hand side and a right hand side. On the left hand side the reactants of the three reactions [1.1] - [3.1] are listed. The right hand side contains the products of the respective reactions.

| Table 1 | | | | | | | | | |
|---|---|---|---|---|---|---|---|---|---|
| $4CO_2$ | $12H_2$ | | | | $4CH_3OH$ | $4H_2O$ | | | |

(continued)

| Table 1 | | | | | | | | | |
|---|---|---|---|---|---|---|---|---|---|
| | | 12H$_2$O | | | | | 12H$_2$ | 6O$_2$ | |
| CO$_2$ | | | 3CH$_4$ | 6O$_2$ | | 6H$_2$O | | | 4CO$_2$ |

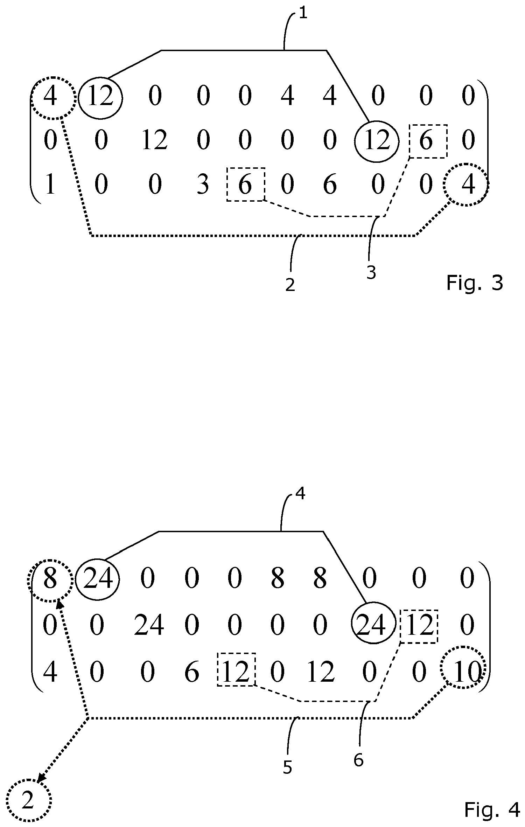

The content of table 1 can also be expressed by the following matrix. This matrix is also shown in Fig. 3. Fig. 3 is used to highlight the mutual interdependence of the underlying processes 103, 106, 114. The link 1 in Fig. 3 shows that 12 mol of hydrogen 107 produced by process 106 are consumed by the process 114. The link 2 shows that 4 mol of CO$_2$ 109 produced by process 103 are consumed by the process 114. The link 3 indicates that 6 mol of O$_2$ 101 produced by process 106 are consumed by the process 103.

$$\begin{pmatrix} 4 & 12 & 0 & 0 & 0 & 4 & 4 & 0 & 0 & 0 \\ 0 & 0 & 12 & 0 & 0 & 0 & 0 & 12 & 6 & 0 \\ 1 & 0 & 0 & 3 & 6 & 0 & 6 & 0 & 0 & 4 \end{pmatrix}$$

If a combustion gas composition CGC with about 60 vol.-% CH4 and 40 vol.-% CO$_2$ is employed, the following equations are valid:

$$8CO_2 + 24H_2 \rightarrow 8CH_3OH + 8H_2O \qquad [1.2]$$

$$24H_2O \text{ (liquid)} \rightarrow 24H_2 + 12O_2 \qquad [2.2]$$

$$6CH_4 + 4CO_2 + 12O_2 \rightarrow 10CO_2 + 12H_2O \qquad [3.2]$$

These equations [1.2] - [3.2] can be rewritten in form of a table (table 2). This table has a left hand side and a right hand side. On the left hand side the reactants of the three reactions [1.2] - [3.2] are listed. The right hand side contains the products of the respective reactions.

| Table 2 | | | | | | | | | |
|---|---|---|---|---|---|---|---|---|---|
| 8CO$_2$ | 24H$_2$ | | | | 8CH$_3$OH | 8H$_2$O | | | |
| | | 24H$_2$O | | | | | 24H$_2$ | 12O$_2$ | |
| 4CO$_2$ | | | 6CH$_4$ | 12O$_2$ | | 12H$_2$O | | | 10CO$_2$ |

The content of table 2 can also be expressed by the following matrix. This matrix is also shown in Fig. 4. Fig. 4 is used to highlight the mutual interdependence of the underlying processes 103, 106, 114. The link 4 in Fig. 4 shows that 24 mol of hydrogen 107 produced by process 106 are consumed by the process 114. The link 5 shows that 8 out of the 10 mol of CO$_2$ 109 produced by process 103 are consumed by the process 114. The link 6 indicates that 12 mol of O$_2$ 101 produced by process 106 are consumed by the process 103.

$$\begin{pmatrix} 8 & 24 & 0 & 0 & 0 & 8 & 8 & 0 & 0 & 0 \\ 0 & 0 & 24 & 0 & 0 & 0 & 0 & 24 & 12 & 0 \\ 4 & 0 & 0 & 6 & 12 & 0 & 12 & 0 & 0 & 10 \end{pmatrix}$$

It is a special advantage of these two process matrices (see Fig. 3 and 4) that the methanol 116 so produced is at least to some extent renewable and that at the same time it is CO$_2$-neutral since CO$_2$ emissions from the clean combustion process 103 are "recycled".

It is a further advantage of the present invention that the oxygen gas 101 from the electrolysis 106 (cf. reaction [2.1] or [2.2]) is used in the two process matrices in order to feed or drive the clean combustion process 103 (reaction [3.1] or [3.2]).

The inventive process matrices represent synergistic processes where all reactants are constituents of a stoichiometrically optimized setup.

It goes without saying that in a practical implementation of the processes of the above matrices certain fluctuations or variations are tolerable. In an ideal or close to ideal embodiment of the invention the molarities of the following table 3 are ensured. The table 3 is to be read as follows: The first line of the table 3 shows that, if in reaction [1.1] 1 mol of $CO_2$ is employed, one has to provide 3 mol $H_2$ in order to produce 1 mol $H_2O$ and 1 mol $CH_3OH$. Note that the bold font is used to highlight in each row the reactants on the left hand side of the respective equation. The last line of the table 3 shows for instance that 1 mol of $O_2$ is employed together with 1/6 mol $CO_2$ plus 1/2 mol $CH_4$ in order to produce 2/3 mol $CO_2$ and 1 mol $H_2O$.

| Table 3 | | | | | | | |
|---|---|---|---|---|---|---|---|
| | Reaction | $O_2$ | $CO_2$ | $H_2O$ | $H_2$ | $CH_4$ | $CH_3OH$ |
| **$CO_2$** | [1.1] | | | 1 | **3** | | 1 |
| **$H_2$** | [1.1] | | **1/3** | 1/3 | | | 1/3 |
| | | | | | | | |
| **$H_2O$** | [2.1] | 1/2 | | | 1 | | |
| | | | | | | | |
| **$CO_2$** | [3.1] | **6** | 4 | 6 | | **3** | |
| **$CH_4$** | [3.1] | **2** | **1/3** and 4/3 | 2 | | | |
| **$O_2$** | [3.1] | | **1/6** and 2/3 | 1 | | **1/2** | |

The inventive process matrix is regarded to be a cogenerating process matrix (see Fig. 3) since it in the first place produces hydrogen 107 in the reaction [2.1] to be used in the reaction [1.1]. This means that in the above table 3 the molarities of hydrogen 107 in equations [2.1] and [1.1] should be the same. Hydrogen 107 is considered to be the first "critical" link (link 1 in Fig. 3) between these two equations. The second "critical" link (link 2 in Fig. 3) is the carbon dioxide 109 provided by the reaction [3.1]. It has to be ensured that this reaction [3.1] provides at least as much carbon dioxide 109 as is required for the reaction of equation [1.1]. Under certain circumstances, the reaction [3.1] might provide more carbon dioxide 109 (see for instance Fig. 4) than required for the reaction [1.1]. The excess carbon dioxide 109 could be stored (e.g. in a buffer tank) or released into the air. There is an implicit third "critical" link (link 3 in Fig. 3). The reaction [2.1] has to provide sufficient oxygen 101 for the clean combustion 103 according to equation [3.1]. It is inherent to the reaction matrices of the invention, that if sufficient hydrogen 107 and carbon dioxide 109 are provided for the reaction [1.1], than sufficient oxygen 101 is provided by the reaction [2.1].

The following table 4 reflects the interdependencies of equations [1.2], [2.2] and [3.2]. Please note that rows 1, 2, 4 of the table 3 and table 4 are identical. All comments which were made in connection with table 3 apply mutates mutandis to table 4.

| Table 4 | | | | | | | |
|---|---|---|---|---|---|---|---|
| | Reaction | $O_2$ | $CO_2$ | $H_2O$ | $H_2$ | $CH_4$ | $CH_3OH$ |
| **$CO_2$** | [1.2] | | | 1 | **3** | | 1 |
| **$H_2$** | [1.2] | | **1/3** | 1/3 | | | 1/3 |
| | | | | | | | |
| **$H_2O$** | [2.2] | 1/2 | | | 1 | | |
| | | | | | | | |
| **$CO_2$** | [3.2] | **3** | 5/2 | 3 | | **3/2** | |
| **$CH_4$** | [3.2] | **2** | **2/3** and 5/3 | 2 | | | |
| **$O_2$** | [3.2] | | **1/3** and 5/6 | 1 | | **1/2** | |

The inventive method for the generation of methanol 116 and for providing output power E5, E7, E8, preferably in the form of heat and/or electric energy, in a plant 100, comprises the following process steps:

- carrying out a water electrolysis process 106 producing oxygen gas 101 and hydrogen gas 107 (this step is carried out by the process module 30),
- providing a combustion gas composition CGC comprising at least 40 vol.-% hydrocarbon gas 102 and at least 25 vol.-% carbon dioxide 117,
- at an input side 201 of a combustion chamber 200, feeding said combustion gas composition CGC and said oxygen gas 101 into the combustion chamber 200,
- maintaining an oxygen-based combustion process 103 for the combustion of the combustion gas composition CGC in said combustion chamber 200 in order to provide output power E8, said combustion process 103 releasing a flue gas 104 at an output side 204 which contains more than 65 vol.-% carbon dioxide 109,
- combining (e.g. by a gas mixing stage 41) said carbon dioxide 109 and said hydrogen gas 107 to form a gas mixture,
- feeding said gas mixture into a catalytic reactor 220,
- in said catalytic reactor 220 carrying out a catalytic process 114 which processes said gas mixture in order to provide methanol 116.

These process steps depend on each other since

- the oxygen gas 101 fed into the combustion chamber 200 is obtained from the water electrolysis 106,
- the carbon dioxide 109 used in the synthesis process 114 is obtained from the flue gas 104 of the combustion process 103,
- the hydrogen gas 107 obtained from the water electrolysis 106 is used for producing the methanol 116.

The synthesis 114 is typically carried out at an increased temperature and pressure in order to be efficient. Synergistic effects can be obtained if in all embodiments a pressurized water electrolysis 106 is employed. The pressurized water electrolysis 106 provides a pressurized hydrogen gas 107 at an output 211. The hydrogen gas 107 typically has a pressure of more than 10 bar at the output 211. This pressurized hydrogen gas 107 can be used to feed the methanol reactor 220. In this case the compressor consumes less energy since it receives at the input side pressurized gas 107. The unit 53 in Fig. 2 might serve as a mixing facility and/or compressor. The unit 53 provides the right stoichiometric mixture or blend and pressure of the gases 107 and 109.

Synergistic effects can also be obtained if delivered energy from one process (e.g. some of the heat E8 of the clean combustion 103) is used to establish the adequate conditions for another process (e.g. the process 106 and/or 114). According to a preferred embodiment of the invention the increased temperature of the flue gas 104 at the output side 204 of the combustion chamber 200 is used to pre-heat or heat the reactants of the reactor 220 since the synthesis 114 is typically carried out at an increased temperature. This principle can be applied to all embodiments.

According to a preferred embodiment of the invention the combustion process 103 provides output power E8 which is used to generate electric energy and heat. At least some of this electric energy and/or heat can be used to energetically support one of the other process steps (e.g. the processes 106 and/or 114).

According to another preferred embodiment of the invention the electric energy E4 which is required to run the water electrolysis 106 is taken from an electric grid (e.g. the grid 411 in Fig. 2) and/or from a renewable source (e.g. from a wind power plant or solar power plant). The plant 100 might comprise a switching or control facility 410 in order to handle the energy supply from and to the electric grid 411. The switching or control facility 410 might comprise an AC-DC converter since the water electrolyzer 210 is supplied by DC current. The double arrow Ex indicates that energy can be taken out of the grid 411 or can be fed into the grid 411.

The process 114 requires relatively pure reactants ($CO_2$ and $H_2$) since there is a risk of weakening the catalyzer inside the reactor 220 by pollutants/contaminations. The feed gas supplied via the feed gas inlet/ring line 221 thus should contain e.g. less than 1 ppm sulfur.

The dashed lines in Fig. 1 and Fig. 2 indicate the flows of media. The respective flows are preferably made switchable or controllable by means of control signal C1, C2 and so forth. Control points, such as valves, shutters, pumps, compressors or other kinds of entities, which enable a software-based control module 300 to reduce or increase a flow or throughput, are employed. The software-based control module 300 issues control signals C1 - C6 to control or switch the control points. Fig. 2 shows arrows placed around the controller 300 to indicate that there are control links which enable the controller 300 to interact with the control points by issuing control signals C1 - C6.

Preferably, the plant 100 of all embodiments comprises a software-based process controller 300, as schematically illustrated in Fig. 2. The software-based process controller 300 is designed and implemented so that it is able to control the flow/supply of at least the two most critical reactants hydrogen 107 and carbon dioxide 109. For this reason the plant 100 comprises at least two control points (e.g. addressed by the signal(s) C2 and C5 and/or C6). The control signals C2 and C6 for instance enable the controller 300 to control the gas mixture provided by the gas mixer 53.

The following table 5 gives further details regarding the control signals of an inventive plant 100. The content of this table 5 is to be understood as an example only.

| Table 5 | | |
|---|---|---|
| control signal | Controls the flow of | Remarks / application example |
| C1 | The supply of electric energy E4 | Could be used to switch the electrolyzer 210 on or off, or to control the operation of the electrolyzer 210 |
| C2 | the hydrogen gas 107 | Could be used to control the hydrogen gas 107 flow |
| C3 | the oxygen gas 101 | Could be used to control the oxygen gas 101 flow |
| C4 | the combustion gas (CG) | Could be used to ensure that the combustion chamber 200 receives the right amount of the combustion gas (CG) |
| C5 | the flue gas 104 | C5 could control an entity for controlling the flue gas 104 emission |
| C6 | the $CO_2$ 109 | Could be used to ensure that the mixer 53 receives the right mixture of $CO_2$ 109 and hydrogen gas 107 |

The control points are connectable to the controller 300. The respective connections are not shown in Fig. 2. The controller 300 preferably comprises an associated parameter storage 301 for the retrieval of stored information and parameters and an input for receiving input signals I1, I2 from other systems. The input signals I1, I2 could come from other systems of the plant 100 or they could come from a grid control facility indicating the load status of the grid 411 and/or the grid frequency.

According to another preferred embodiment of the invention the controller 300 is employed in order to contribute to an equalization of load fluctuations of the electric grid 411 and/or to the frequency control of the electric grid 411. For this purpose the software-based process controller 300 is designed and implemented so that it is able to control the energy output E8 of the combustion process 103 and/or the energy consumption E4 of the water electrolysis 106 so as to contribute to the load equalization and/or the frequency control of the electric grid 411. Furthermore, an immediate shut-off of the water electrolyzer 210 (e.g. using a control signal for control signal C1) offers the respective load reserve for the grid 411.

According to another preferred embodiment of the invention the controller 300 is employed in order to control the flow of gases and reactants (e.g. via the control signals mentioned) so that the methanol reactor 220 is operated at a load of more than 80 % and preferably at a load of close to 100 %.

According to another preferred embodiment the plant 100 (cf. Fig. 2) is specifically designed for the generation of output power in the form of electric energy and heat, and for the production of methanol 116. The apparatus 100 comprises

- a water electrolysis facility 210 which can be supplied with electric (DC) energy E4 and water 105. The water electrolysis facility 210 is designed in order to produce hydrogen gas 107 and oxygen gas 101. The water electrolysis facility 210 comprises a hydrogen gas outlet 211 and an oxygen gas outlet 212.
- a combustion chamber 200 (e.g. being part of an thermal engine) designed for maintaining an oxygen-based combustion process 103 in order to provide output power E8. The combustion chamber 200 comprises an input side 201, and a flue gas outlet 204 for releasing a flue gas 104 which contains more than 65 vol.-% carbon dioxide 109.
- a gas connection 250 for feeding the oxygen gas 101 from the oxygen gas outlet 212 to the input side 201 of the combustion chamber 200,
- a gas connection 202 for feeding a combustion gas composition CGC comprising a hydrocarbon gas 102 (e.g. methane 102) and carbon dioxide 117 to the input side 201 of the combustion chamber 200.
- a gas mixer 41, 53 for providing a gas mixture. The gas mixer 41, 53 is connectable to the hydrogen gas outlet 211 and directly or indirectly connectable to the flue gas outlet 204.
- a catalytic reactor 220 for carrying out a catalytic process 114 which processes the gas mixture in order to provide methanol 116.

Details of a suitable methanol reactor 220 are disclosed and claimed in the international patent application PCT/EP2010/064948, which is currently assigned to the applicant of the present application.

A combined heat and power plant (CHP) for the purposes of the present invention is a thermal engine or a power station designed to simultaneously generate both electricity and useful heat (here called output power). The CHP captures some or all of the by-product heat for heating purposes.

The plant 100 in one embodiment comprises a gas turbine CHP plant 400 which uses the waste heat in the flue gas 104 of the gas turbine. The combustion gas composition CGC is used as gaseous fuel for "firing" the gas turbine.

The plant 100 in another embodiment comprises a gas engine CHP plant 400 which uses a reciprocating gas engine. The combustion gas composition CGC is used as gaseous fuel for "firing" the reciprocating gas engine.

The plant 100 in another embodiment comprises a biofuel engine CHP plant 400 which employs an adapted reciprocating gas engine or gas diesel engine.

All embodiments of the invention might comprise means for biogas upgrading or means for performing a purification process. The upgrading or purification can be designed so as to remove or reduce undesired contaminations, such as $H_2S$. The upgrading or purification can be designed to reduce the $CO_2$ content in cases where too much $CO_2$ is contained in the biogenic gas. Typically, a water washing system is employed where the biogenic gas is guided through a water scrubber. The water absorbs $CO_2$ and the gas emitted by the washing system has a reduced $CO_2$ vol.-%. In cases where the $CO_2$ is ideal or close to ideal, but where other contaminations are to be removed, one could use a water washing system where the water is saturated with $CO_2$. The saturated water does not absorb any further $CO_2$, and the $CO_2$ content of the biogenic gas guided through this washing system remains essentially constant. The water washing system is employed to wash out some of the undesired by-products.

Reference number listing:

| | |
|---|---|
| links | 1, 2, 3, 4, 5, 6 |
| | |
| process modules | 30, 40, 50 |
| | |
| mixing process | 41 |
| | |
| gas mixer | 53 |
| | |
| plant | 100 |
| Oxygen gas | 101 |
| gaseous hydrocarbon (methane) | 102 |
| "clean" combustion | 103 |
| Flue gas | 104 |
| water | 105 |
| Hydrogen gas | 107 |
| Electrolysis process | 106 |
| Carbon dioxide | 109 |
| Excess water | 110 |
| Separation process | 111 |
| Methanol synthesis | 114 |
| Methanol-water mixture | 115 |
| Methanol | 116 |
| Carbon dioxide | 117 |
| | |
| Dashed lines (gas supply lines) | 121, 122 |
| | |
| Combustion chamber of an internal combustion engine | 200 |
| input side | 201 |

(continued)

| | |
|---|---|
| combustion gas composition infeed | 202 |
| oxygen gas infeed | 203 |
| flue gas outlet | 204 |
| water separator | 205 |
| carbon dioxide outlet | 206 |
| gas-oxygen mixer or gas-oxygen-$CO_2$ mixer | 207 |
| | |
| water electrolysis facility | 210 |
| hydrogen output | 211 |
| oxygen output | 212 |
| Water supply (tap) | 213 |
| | |
| methanol reactor | 220 |
| methanol outlet | 222 |
| feed gas inlet / ring line | 221 |
| | |
| gas connection | 250 |
| gas connections | 251, 252 |
| gas connection | 253 |
| gas feedback | 254 |
| | |
| software-based process controller | 300 |
| parameter storage | 301 |
| | |
| combined heat and power plant (CHP) | 400 |
| | |
| Switch and control facility | 410 |
| electric grid | 411 |
| | |
| Control signals | C1, C2, C3, C4, C5, C6, ... |
| combustion gas composition | CGC |
| electric energy | Ex |
| Energy consumption | E4 |
| Energy output | E5 |
| Energy consumption | E6 |
| Energy output | E7 |
| Energy output | E8 |
| input signals | I1, I2, ... |

**Claims**

1. Method for the generation of methanol (116) and for providing output power (E5, E7, E8) in a plant (100), comprising the process steps:

   - carrying out a water electrolysis process (106) producing oxygen gas (101) and hydrogen gas (107),
   - providing a combustion gas composition (CGC) comprising at least 40 vol.-% hydrocarbon gas (102) and at least 25 vol.-% carbon dioxide (117),
   - at an input side (201) of a combustion chamber (200), feeding said combustion gas composition (CGC) and said oxygen gas (101) into the combustion chamber (200),
   - maintaining an oxygen-based combustion process (103) for the combustion of the combustion gas composition (CGC) in said combustion chamber (200) in order to provide output power (E8), said combustion process (103) releasing a flue gas (104) at an output side (204) which contains more than 65 vol.-% carbon dioxide (109),
   - combining (41) said carbon dioxide (109) and said hydrogen gas (107) to form a gas mixture,
   - feeding said gas mixture into a catalytic reactor (220),
   - in said catalytic reactor (220) carrying out a catalytic process (114) which processes said gas mixture in order to provide methanol (116), wherein
   at least part of said flue gas (104) or of said carbon dioxide (109) is fed back from said output side (204) to said input side (201) of the combustion chamber (200) in order to increase the amount of carbon dioxide (109) at the input side (201), and wherein means (111, 205) for removing water ($H_2O$) from said flue gas (104) are employed.

2. The method of claim 1, wherein said output power (E5, E7, E8) is provided in the form of heat and/or electric energy.

3. The method of claim 1 or 2, wherein said combustion chamber (200) is part of a gas Otto engine, a gas diesel engine, a gas turbine or a combined heat and power plant (400).

4. The method of claim 1 or 2, wherein said combustion chamber (200) is part of a combustion engine (400) which comprises at least two cylinders and wherein only a part of the cylinders of said combustion engine (400) are fed with said oxygen gas (101).

5. The method of claim 1, 2, 3 or 4, wherein a cooling liquid (115) comprising methanol and water is injected or sprayed into the gas flow prior the combustion chamber (200) for cooling purposes.

6. The method of claim 1 or 2, wherein a cooling liquid (115) comprising methanol and water is combined with or injected or sprayed into said part (254) of said flue gas (104) which is fed back from said output side (204) to said input side (201) of the combustion chamber (200).

7. The method according to one of the claims 1 through 7, wherein said combustion gas composition (CGC) comprises less than 75 vol.-% methane (102).

8. The method according to one of the claims 1 through 7, wherein said combustion gas composition (CGC) comprises biogenic gas.

9. The method of claim 1, 2 or 3, wherein at least some of the process steps of claim 1 depend on each other since

   - the oxygen gas (101) fed into the combustion chamber (200) is obtained from the water electrolysis process (106),
   - the carbon dioxide (109) used in the catalytic process (114) is contained in said flue gas (104) of the combustion process (103),
   - the hydrogen gas (107) used in the catalytic process (114) is obtained from the water electrolysis process (106), and wherein at least the mass flows of the carbon dioxide (109) and the hydrogen gas (107) are controlled so as to be close to a ratio of 1 mol $CO_2$ versus 3 mol of $H_2$ or to exactly have a ratio of 1 mol $CO_2$ versus 3 mol of $H_2$.

10. The method of claim 9, wherein output power (E8) in the form of heat produced by said combustion chamber (200) is used to energetically support or supply one or more of the following process steps:

   - said catalytic process (114), and/or

- said water electrolysis process (106).

11. Plant (100) for the generation of methanol (116) and for providing output power (E5, E7, E8) comprising:

  - a water electrolysis facility (210) which can be supplied by electric energy (E4) and water (105) and being designed in order to produce hydrogen gas (107) and oxygen gas (101), the water electrolysis facility (210) comprising a hydrogen gas outlet (211) and an oxygen gas outlet (212),
  - a thermal engine with at least one combustion chamber (200) designed for maintaining an oxygen-based combustion process (103) in order to provide output power (E8), said combustion chamber (200) comprising

    ∘ an input side (201), and
    ∘ a flue gas outlet (204) for releasing a flue gas (104) which contains more than 65 vol.-% carbon dioxide (109),

  - a gas connection (250) for feeding said oxygen gas (101) from said oxygen gas outlet (212) to the input side (201) of the combustion chamber (200),
  - a gas connection (202) for feeding a combustion gas composition (CGC) comprising a hydrocarbon gas (102) and carbon dioxide (117) to the input side (201) of the combustion chamber (200),
  - a gas mixer (41; 53) for providing a gas mixture, said gas mixer (41; 53) being connectable to said hydrogen gas outlet (211) and being directly or indirectly connectable to said flue gas outlet (204),
  - a catalytic reactor (220) for carrying out a catalytic process (114) which processes said gas mixture in order to provide said methanol (116),
  - a combustion gas-oxygen mixer or a gas-oxygen-$CO_2$ mixer (207) at the input side (201) of the combustion chamber (200),
  - further comprising means (111, 205) for removing water ($H_2O$) from said flue gas (104).

12. The plant (100) of claim 11, wherein said catalytic reactor (220) comprises:

  - a methanol outlet (222), and
  - a feed gas inlet (221) for feeding said gas mixture into the catalytic reactor (220).

13. The plant (100) of claim 11, wherein said catalytic reactor (220) comprises a ring line serving as feed gas inlet (221).

14. The plant (100) of claim 11, 12 or 13 further comprising means (111; 205) for removing micro elements from said flue gas (104).

15. The plant (100) of claim 11, 12 or 13 comprising a gas Otto engine or a gas diesel engine having at least two combustion chambers (200).

16. The plant (100) of claim 11, 12 or 13 comprising a software-based control module (300) for controlling by means of control signals (C1, C2, ...) the flow or gases inside the plant (100).

17. The plant (100) of claim 11, wherein said output power (E5, E7, E8) is provided in the form of heat and/or electric energy.

**Patentansprüche**

1. Verfahren für die Erzeugung von Methanol (116) und zum Bereitstellen von Ausgabeleistung (E5, E7, E8), in einer Anlage (100), umfassend die Verfahrensschritte:

  - Durchführen einer Wasser-Elektrolyse Verfahrens (106), das Sauerstoffgas (101) und Wasserstoffgas (107) produziert,
  - Bereitstellen einer Verbrennungsgaszusammensetzung (CGC), die mindestens 40 Vol.-% Kohlenwasserstoffgas (102) und mindestens 25 Vol.-% Kohlendioxid (117) umfasst,
  - an einer Eingangsseite (201) einer Brennkammer (200) Einspeisen der Verbrennungsgaszusammensetzung (CGC) und das Sauerstoffgas (101) in die Brennkammer (200),
  - Aufrechterhalten eines sauerstoffbasierten Verbrennungsverfahrens (103) für die Verbrennung der Verbrennungsgaszusammensetzung (CGC) in der Brennkammer (200), um Ausgabeleistung (E8) bereit zu stellen, wobei dieses Verbrennungsverfahren (103) ein Rauchgas (104) an einer Ausgangsseite (204) freisetzt, die mehr als 65 Vol.-% Kohlendioxid (109) enthält,
- Kombinieren (41) des Kohlendioxids (109) und des Wasserstoffgases (107), um eine Gasmischung zu bilden,
- Einspeisen der Gasmischung in einen katalytischen Reaktor (220),
- Durchführen eines katalytischen Verfahrens (114) in dem katalytischen Reaktor (220), der die Gasmischung verarbeitet, um Methanol (116) bereit zu stellen, worin mindestens ein Teil des Rauchgases (104) oder des Kohlendioxids (109) von der Ausgangsseite (204) zurückgeführt wird zu der Eingansseite (201) der Brennkammer (200), um die Menge des Kohlendioxids (109) an der Eingansseite (201) zu erhöhen, und worin Mittel (111, 205) zum Entfernen von Wasser ($H_2O$) aus dem Rauchgas (104) eingesetzt werden.

2. Das Verfahren des Anspruchs 1, worin die Ausgabeleistung (E5, E7, E8) in Form von Wärme und/oder elektrischer Energie bereitgestellt wird.

3. Das Verfahren des Anspruchs 1 oder 2, worin die Brennkammer (200) Teil eines Gas-Otto-Motors, eines Gas-Diesel-Motors, einer Gas Turbine oder einer kombinierten Wärme und Energieanlage (400) ist.

4. Das Verfahren des Anspruchs 1 oder 2, worin die Brennkammer (200) Teil eines Verbrennungsmaschine (400) ist, die mindestens zwei Zylinder umfasst und worin nur ein Teil der Zylinder der Verbrennungsmaschine (400) mit dem Sauerstoffgas (101) beschickt werden.

5. Das Verfahren der Ansprüche 1 2, 3 oder 4, worin eine Kühlflüssigkeit (115), die Methanol und Wasser umfasst, in den Gasfluß vor der Brennkammer (200) für Kühlzwecke eingespritzt oder gesprüht wird.

6. Das Verfahren des Anspruchs 1 oder 2, worin eine Kühlflüssigkeit (115), die Methanol und Wasser umfasst, kombiniert wird mit, oder eingespritzt oder gesprüht wird in den Teil (254) des Rauchgases (104), der von der Ausgangsseite (204) zurück geführt wird zu der Eingangsseite (201) der Brennkammer (200).

7. Das Verfahren nach einem der Ansprüche 1 bis 7, worin die Verbrennungsgaszusammensetzung (CGC) weniger als 75 Vol.-% Methan (102) umfasst.

8. Das Verfahren nach einem der Ansprüche 1 bis 7, worin die Verbrennungsgaszusammensetzung (CGC) biogenes Gas umfasst.

9. Das Verfahren des Anspruchs 1, 2 oder 3, worin mindestens einige der Verfahrensschritte des Anspruchs 1 voneinander abhängen, da

- das Sauerstoffgas (101), das in die Brennkammer gespeist wird, aus dem Wasser-Elektrolyse Verfahren (106) stammt,
- das Kohlendioxid (109), das in dem katalytischen Prozess (114) verwendet wird, in dem Rauchgas (104) des Verbrennungsverfahrens (103) enthalten ist,
- das Wasserstoffgas (107), das in dem katalytischen Prozess (114) verwendet wird, aus dem Wasser-Elektrolyse Verfahren (106) stammt,
und worin mindestens die Massenflüsse des Kohlendioxids (109) und des Wasserstoffgases (107) so kontrolliert werden, dass sie nahe an einem Verhältnis von 1 Mol $CO_2$ zu 3 Mol $H_2$ oder exakt ein Verhältnis von 1 Mol $CO_2$ zu 3 Mol $H_2$ haben.

10. Das Verfahren des Anspruchs 9, worin Ausgabeleistung (E8), die in Form von Wärme durch die Brennkammer (200) produziert wird, verwendet wird, um energetisch einen oder mehrere der folgenden Verfahrensschritte zu unterstützen oder zu versorgen:

- der katalytische Prozess (114), und/oder
- das Wasser-Elektrolyse Verfahren (106).

11. Anlage (100) zum Erzeugen von Methanol (116) und zum Bereitstellen von Ausgabeleistung (E5, E7, E8) umfassend:

- eine Wasser-Elektrolyse-Einrichtung (210), die mit elektrischer Energie (E4) und Wasser (105) versorgt werden kann und die ausgelegt ist, um Wasserstoffgas (107) und Sauerstoffgas (101) zu produzieren, wobei die Wasser-Elektrolyse-Einrichtung (210) einen Wasserstoffgas-Auslass (211) und einen Sauerstoffgas-Auslass (212) umfasst,

- eine Wärmemaschine mit mindestens einer Brennkammer (200), die dazu ausleget ist ein sauerstoff-basiertes Verbrennungsverfahren (103) aufrecht zu erhalten, um Ausgabeleistung (E8) bereit zu stellen, wobei die Brennkammer (200) umfasst

  ◦ eine Eingangsseite (201), und
  ◦ einen Rauchgas-Ausgang (204) zum Freisetzen von Rauchgas (104), das mehr als 65 Vol.-% Kohlendioxid (109) enthält,

- eine Gasverbindung (250) zum Speisen des Sauerstoffgases (101) von dem Sauerstoffgas-Auslass (212) zu der Eingangsseite (201) der Brennkammer (200),
- eine Gasverbindung (202) zum Speisen des einer Verbrennungsgaszusammensetzung (CGC), die Kohlenwasserstoffgas (102) und Kohlendioxid (117) umfasst, zu der Eingangsseite (201) der Brennkammer (200),
- einen Gasmischer (41; 53) zum Bereitstellen einer Gasmischung, wobei der Gasmischer (41; 53) verbindbar ist mit dem Wasserstoffgas-Auslass (211) und direkt oder indirekt verbindbar ist mit dem Rauchgas-Ausgang (204),
- einen katalytischen Reaktor (220) zum Durchführen eines katalytischen Verfahrens (114), das die Gasmischung verarbeitet um Methanol (116) bereit zu stellen,
- einen Verbrennungsgas-Sauerstoff-Mischer oder einen Gas-Sauerstoff-$CO_2$-Mischer (207) an der Eingangsseite (201) der Brennkammer (200),
- weiterhin umfassend Mittel (11, 205) zum Entfernen von Wasser ($H_2O$) aus dem Rauchgas (104).

12. Die Anlage (100) des Anspruchs 11, worin der katalytischen Reaktor (220) umfasst:

    - einen Methanol-Auslass (222) und
    - einen Gasspeiseeingang (221) zum Speisen der Gasmischung in den katalytischen Reaktor (220).

13. Die Anlage (100) des Anspruchs 11, worin der katalytischen Reaktor (220) eine Ringleitung umfasst, die als Gasspeiseeingang (221) dient.

14. Die Anlage (100) des Anspruchs 11, 12 oder 13, die zusätzlich Mittel (111; 205) zum Entfernen von Mikroelementen aus dem Rauchgas (104) umfasst.

15. Die Anlage (100) des Anspruchs 11, 12 oder 13, die einen Gas-Otto-Maschine oder eine Gas-Diesel-Maschine umfasst, die mindestens zwei Brennkammern (200) hat.

16. Die Anlage (100) des Anspruchs 11, 12 oder 13, die ein software-basiertes Kontrollmodul (300) zum Kontrollieren der Flüsse oder Gase in der Anlage (100) mittels Kontrollsignalen (C1, C2, ...) umfasst.

17. Die Anlage (100) des Anspruchs 11, worin die Ausgabeleistung (E5, E7, E8) in Form vom Wärme und/oder elektrischer Energie bereit gestellt wird.

**Revendications**

1. Procédé pour produire du méthanol (116) et pour fournir une puissance de sortie (E5, E7, E8) dans une installation (100) comprenant les étapes suivantes :

   - réaliser un processus d'électrolyse de l'eau (106) produisant du gaz oxygène (101) et du gaz hydrogène (107),
   - fournir une composition de gaz de combustion (CGC) comprenant au moins 40 % en volume de gaz hydrocarbure (102) et au moins 25 % en volume de dioxyde de carbone (117),
   - sur un côté entrée (201) d'une chambre de combustion (200), amener ladite composition de gaz de combustion (CGC) et ledit gaz oxygène (101) dans la chambre de combustion (200),
   - maintenir un processus de combustion à base d'oxygène (103) pour la combustion de la composition de gaz de combustion (CGC) dans ladite chambre de combustion (200) pour fournir une puissance de sortie (E8), ledit processus de combustion (103) libérant un gaz de combustion (104) sur un côté sortie (204) qui contient plus de 65 % en volume de dioxyde de carbone (109),
   - combiner (41) ledit dioxyde de carbone (109) et ledit gaz hydrogène (107) pour former un mélange de gaz,

- amener ledit mélange de gaz dans un réacteur catalytique (220),
- dans ledit réacteur catalytique (220) effectuer un procédé catalytique (114) qui transforme ledit mélange de gaz pour fournir du méthanol (116), dans lequel au moins une partie dudit gaz de combustion (104) ou dudit dioxyde de carbone (109) est ramené depuis ledit côté sortie (204) audit côté entrée (201) de la chambre de combustion (200) pour augmenter la quantité de dioxyde de carbone (109) sur le côté entrée (201), et dans lequel il est utilisé des moyens (111, 205) pour retirer l'eau ($H_2O$) dudit gaz de combustion (104).

2. Procédé selon la revendication 1, dans lequel ladite puissance de sortie (E5, E7, E8) est fournie sous forme de chaleur et/ou d'énergie électrique.

3. Procédé selon la revendication 1 ou 2, dans lequel ladite chambre de combustion (200) fait partie d'un moteur Otto à gaz, d'un moteur diesel à gaz, d'une turbine à gaz ou d'une centrale de production combinée chaleur-électricité (400).

4. Procédé selon la revendication 1 ou 2, dans lequel ladite chambre de combustion (200) fait partie d'un moteur à combustion (400) qui comprend au moins deux cylindres, et dans lequel seule une partie des cylindres dudit moteur à combustion (400) est alimentée avec ledit gaz oxygène (101).

5. Procédé selon la revendication 1, 2, 3 ou 4, dans lequel un liquide de refroidissement (115) comprenant du méthanol et de l'eau est injecté ou pulvérisé dans le flux de gaz avant la chambre de combustion (200) à des fins de refroidissement.

6. Procédé selon la revendication 1 ou 2, dans lequel un liquide de refroidissement (115) comprenant du méthanol et de l'eau est combiné avec ou injecté ou pulvérisé dans ladite partie (254) dudit gaz de combustion (104) qui est ramenée depuis le côté sortie (204) audit côté entrée (201) de la chambre de combustion (200).

7. Procédé selon l'une des revendications 1 à 7, dans lequel ladite composition de gaz de combustion (CGC) comprend moins de 75 % en volume de méthane (102).

8. Procédé selon l'une des revendications 1 à 7, dans lequel ladite composition de gaz de combustion (CGC) comprend du gaz biogénique.

9. Procédé selon la revendication 1, 2 ou 3, dans lequel au moins certaines des étapes du procédé de la revendication 1 dépendent les unes des autres dans la mesure où

- le gaz oxygène (101) amené dans la chambre de combustion (200) est obtenu par le processus d'électrolyse de l'eau (106),
- le dioxyde de carbone (109) utilisé dans le procédé catalytique (114) est contenu dans ledit gaz de combustion (104) du procédé de combustion (103),
- le gaz hydrogène (107) utilisé dans le procédé catalytique (114) est obtenu par le processus d'électrolyse de l'eau (106),
et dans lequel au moins les débits massiques du dioxyde de carbone (109) et du gaz hydrogène (107) sont contrôlés de façon à être proches d'un rapport de 1 mole de $CO_2$ pour 3 moles de $H_2$, ou pour avoir exactement le rapport 1 mole de $CO_2$ pour 3 moles de $H_2$.

10. Procédé selon la revendication 9, dans lequel la puissance de sortie (E8) sous forme de chaleur produite par ladite chambre de combustion (200) est utilisée pour fournir un soutien énergétique ou pour réaliser une ou plusieurs des étapes de procédé suivantes :

- ledit procédé catalytique (114), et/ou
- ledit processus d'électrolyse de l'eau (106).

11. Installation (100) pour la production de méthanol (116) et pour la fourniture d'une puissance de sortie (E5, E7, E8) comprenant :

- une installation d'électrolyse de l'eau (210) qui peut être alimentée par de l'énergie électrique (E4) et de l'eau (105) et qui est conçue pour produire du gaz hydrogène (107) et du gaz oxygène (101), l'installation d'électrolyse de l'eau (210) comprenant une sortie de gaz hydrogène (211) et une sortie de gaz oxygène (212),

- un moteur thermique avec au moins une chambre de combustion (200) conçu pour maintenir un procédé de combustion à base d'oxygène (103) pour fournir une puissance de sortie (E8), ladite chambre de combustion (200) comprenant

  • un côté entrée (201), et
  • une sortie de gaz de combustion (204) pour libérer un gaz de combustion (104) qui contient plus de 65 % en volume de dioxyde de carbone (109),

- un raccord de gaz (250) pour amener ledit gaz oxygène (101) depuis ladite sortie de gaz oxygène (212) au côté entrée (201) de ladite chambre de combustion (200),
- un raccord de gaz (202) pour amener une composition de gaz de combustion (CGC) comprenant un gaz hydrocarbure (102) et du dioxyde de carbone (117) au côté entrée (201) de la chambre de combustion (200),
- un mélangeur de gaz (41 ; 53) pour fournir un mélange de gaz, ledit mélangeur de gaz (41 ; 53) pouvant être raccordé à ladite sortie de gaz hydrogène (211) et pouvant être raccordé directement ou indirectement à ladite sortie de gaz de combustion (204),
- un réacteur catalytique (220) pour effectuer un procédé catalytique (114) qui transforme ledit mélange de gaz pour fournir ledit méthanol (116),
- un mélangeur gaz de combustion-oxygène ou un mélangeur gaz-oxygène-$CO_2$ (207) sur le côté entrée (201) de la chambre de combustion (200),
- comprenant en outre des moyens (111, 205) pour retirer l'eau ($H_2O$) dudit gaz de combustion (104).

12. Installation (100) selon la revendication 11, dans laquelle ledit réacteur catalytique (220) comprend :

  - une sortie de méthanol (222), et
  - une entrée de gaz d'alimentation (221) pour amener ledit mélange de gaz dans le réacteur catalytique (220).

13. Installation (100) selon la revendication 11, dans laquelle ledit réacteur catalytique (220) comprend une conduite circulaire servant d'entrée de gaz d'alimentation (221).

14. Installation (100) selon la revendication 11, 12 ou 13, comprenant en outre des moyens (111 ; 205) pour retirer les microéléments dudit gaz de combustion (104).

15. Installation (100) selon la revendication 11, 12 ou 13, comprenant un moteur Otto à gaz ou un moteur diesel à gaz ayant au moins deux chambres de combustion (200).

16. Installation (100) selon la revendication 11, 12 ou 13 comprenant un module de contrôle logiciel (300) pour contrôler, par le biais de signaux de contrôle (C1, C2, ...) le flux ou les gaz à l'intérieur de l'installation (100).

17. Installation (100) selon la revendication 11, dans laquelle ladite puissance de sortie (E5, E7, E8) est fournie sous forme de chaleur et/ou d'énergie électrique.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2010067812 W
- DE 102009007567 A1
- WO 0025380 A2
- US 5342702 A
- WO 9531423 A
- WO 2008012039 A
- US 6148602 A
- EP 2010064948 W

### Non-patent literature cited in the description

- Beyond Oil and Gas: The Methanol Economy. *Angewandte Chemie Int. Ed.,* 2005, vol. 44, 2636-2639
- **G.A. OLAH et al.** Chemical Recycling of Carbon Dioxide to Methanol and Dimethyl Ether: From Greenhouse Gas to Renewable, Environmentally Carbon Neutral Fuels and Synthetic Hydrocarbons. *Journal of Organic Chemistry,* 2009, vol. 74 (2), 487-498